# EUROPEAN PATENT APPLICATION

(11) **EP 3 533 861 A1**
(43) Date of publication of application: **04.09.2019**
(21) Application number: 18159775.8
(22) Date of filing: 02.03.2018
(51) Int. Cl.: C12N 1/04, C12N 1/20, A23L 33/135, A23C 9/12

(54) **PROCESS FOR LYOPHILIZING A MICROORGANISM**

(71) Applicant: DuPont Nutrition Biosciences ApS, 1411 Copenhagen K (DK)
(72) Inventor: HENRI, Erwan, 75017 Paris (FR); BABIN, Geoffrey, 75017 Paris (FR); BOULANGER, Geoffrey, 75017 Paris (FR); ANGOUMOIS, Yann, 75017 Paris (FR); NORMAND, Nicolas, 75017 Paris (FR); CHAIZE, Jean-Noël, 75017 Paris (FR); PERIGNON, Alexia, 75017 Paris (FR)
(74) Representative: DuPont EMEA

(57) **Abstract**

The invention provides an improved method for lyophilising lactic bacteria of the *coccus* type, and freeze-dried bacterial compositions resulting from the method.

## Description

### Field of Invention

The present invention relates to the field of lyophilized microorganisms.

### Background of the Invention

Freeze-drying microorganisms (lyophilization) is a very well-established method for preparing them for long-term storage. The approaches used vary, but they all share the basic process associated with lyophilization, namely freezing of the sample, application of a high vacuum, warming of the sample while under vacuum which causes water sublimation, driving off excess water through a drying phase, releasing the vacuum, and finally sealing of the sample to prevent water uptake. This general process is used to preserve bacteria, fungi, yeasts, proteins, nucleic acids, and any other molecules which may be degraded due to the presence of water.

Preserving bacteria by lyophilization requires that the bacteria be suspended in a medium that helps to maintain their viability through freezing, water removal, and subsequent storage. The ideal solution will have a component that helps to form a solid "cake" which gives body to the bacterial suspension once freeze-dried.

Freeze-drying is best performed on healthy, actively growing cells which are collected and suspended in freeze-drying medium. Cells are usually cultured in liquid medium, and then collected by concentration, such as by ultrafiltration.

A basic freeze-drying process can be divided into three stages: freezing, primary drying, and secondary drying.

### Freezing

Freezing is typically carried out to bring the microbial suspension to about - 40°C.

### Primary drying

Primary drying removes readily available frozen water. Once a sample is frozen, a vacuum is applied, typically at a pressure under 25 Pa. During primary drying the temperature of the sample is raised to create sufficient molecular motion to allow water molecules to sublime, i.e., go from solid ice to gas, as long as a vacuum is present. Ideally water is removed faster than the sample absorbs heat. The sublimation of the water thus keeps the sample frozen. If the sample increases in temperature too rapidly, it will melt which decreases the value of freeze-drying.

The use of matrix forming agents, such as BSA or mannitol, is very useful for helping to form a frozen sample that maintains its shape as water is removed.

Primary drying can take anywhere from 3-4 hours for a small sample to overnight for a fully loaded shelf freeze-dryer.

### Secondary drying

Secondary drying forces out residual water by increasing the temperature of the sample. In shelf dryers, the samples can be increased to 20°C for several hours prior to sealing. Freeze-drying with a basic system usually does not allow a separation between primary and secondary drying. As the frozen water is driven off from the sample in primary drying, its temperature will rise to match the ambient temperature. This period of ambient drying will serve as a secondary drying phase. Following secondary drying, vials and/or ampoules are sealed.

The freeze-drying process is time consuming, adding to the cost of industrially produced freeze-dried microbes. There is a need to improve existing freeze-drying processes to increase the rate of water removal, thus increasing the throughput.

### Summary of the Invention

In a first aspect, the invention provides a freeze-dried bacterial composition comprising:
(1) freeze-dried Lactic bacteria of the *coccus* type;
(2) at least one hydrocolloid.

In a second aspect, the invention provides a method for preparing lyophilized Lactic bacteria of the *coccus* type, comprising the step of adding a hydrocolloid to an aqueous suspension of Lactic bacteria of the *coccus* type before freezing.

In a third aspect, the invention provides a freeze-dried bacterial composition obtainable or obtained by the method of the invention.

### Detailed Description of the Invention

All documents referred to herein are incorporated by reference.

The inventors have surprisingly found that water removal during lyophilization of Lactic bacteria of the *coccus* type is more efficient and/or accelerated if a hydrocolloid is added to an aqueous suspension of the bacteria prior to freezing.

For the purposes of the invention the expression *coccus* means any bacterium that has a spherical, ovoid, or generally round shape. It is one of the three distinct bacterial shapes, the other two being bacillus (rod-shaped) and spiral-shaped cells.

### Process/method of the invention

The invention provides an improvement to conventional lyophilization. The improvement being: in a method for the preparation of lyophilized Lactic bacteria of the *coccus* type, comprising the steps of:
(1) providing an aqueous suspension of Lactic bacteria of the *coccus* type;
(2) freezing the aqueous suspension in a form suitable for lyophilization to form a frozen suspension; and
(3) subjecting the frozen suspension to a vacuum suitable for removal of water by sublimation,
characterized in that a hydrocolloid is added to the aqueous suspension prior to freezing.

The method of the invention is suitable for suspensions containing one species and/or strain of Lactic bacteria of the *coccus* type, a mixture of species and/or strains of Lactic bacteria of the *coccus* type. In one aspect the suspension contains one species Lactic bacteria of the *coccus* type, and, optionally, one strain of Lactic bacteria of the *coccus* type. In one aspect the suspension contains a mixture of species of Lactic bacteria of the *coccus* type, and optionally a mixture of strains of Lactic bacteria of the *coccus* type. In one aspect the suspension contains a mixture of species of Lactic bacteria of the *coccus* type.

In a further preferred embodiment the Lactic bacteria of the *coccus* type are selected from *Streptococcus, Lactococcus, Enterococcus, Oenococcus* and *Pediococcus,* and mixtures thereof. In a particularly preferred embodiment the bacteria are selected from *Streptococcus thermophilus, Lactococcus lactis* (in particular subspecies *cremoris), Lactococcus lactis lactis* and mixtures thereof.

In one preferred embodiment the Lactic bacteria of the *coccus* type, is selected from *Streptococcus thermophilus* (ST11688 strain), *Lactococcus lactis ssp. cremoris* (SC0108 strain), and mixtures thereof.

The invention involves the addition of a hydrocolloid, optionally with a cryoprotectant, such as a sugar, preferably sucrose, to an aqueous suspension of Lactic bacteria of the *coccus* type prior to freeze-drying. Preferably the addition of hydrocolloid is made to a suspension of bacteria in which the bacteria are present at between 50 g/kg and 200 g/kg, more preferably between 70 g/kg to 150 g/kg, based on dry weight. Preferably the addition of hydrocolloid takes place after fermentation is complete.

For the purposes of the invention, a hydrocolloid is defined as a biopolymer having hydroxyl groups, that yields a viscous dispersion or a gel when dispersed in water. Examples of suitable hydrocolloids are thickeners and gelling agents. Thickeners exhibit non-specific entanglement of conformationally disordered polymer chains, resulting in thickening of a water solution or dispersion. Examples of thickeners are alginate, xanthan, carboxymethyl cellulose, methyl cellulose and hydroxypropyl cellulose, guar, locust bean gum, Konjac maanan, gum tragacanth, cellulose gum, and vegetable starches such as potato starch, corn starch, tapioca starch, wheat starch and cassava starch.

Gelling agents are capable of association or cross-linking of the polymer chains to form a three dimensional network that traps or immobilises the water within it to form a rigid structure that is resistant to flow. Examples of gelling agents include modified starch, agar, carrageenan, pectin, gellan gum and gelatin.

The hydrocolloid is added as a solid or as an aqueous solution or suspension to an aqueous suspension of the *coccus* bacteria, optionally with a cryoprotectant, such as a sugar, preferably sucrose. The addition preferably takes place at or below 10°C, more preferably at or below 5°C. The hydrocolloid is not particularly limited, although it is preferred to use hydrocolloids that are water soluble and/or dissolvable and/or dispersible at temperatures below 10°C, more preferably below 5°C because it is desirable to avoid excessive heating of the bacterial suspension, which would result in undesired metabolic activity of the bacteria. For this reason, thickeners are preferred over gelling agents, as most thickeners are soluble and/or dispersible at low temperature.

Particularly preferred hydrocolloids are guar, xanthan, cellulose gum, alginate, locust bean gum (carob bean gum) and starch, in particular potato starch, gelatin, in particular porcine or bovine gelatin, and mixtures of any of these. Particularly preferred hydrocolloids of the thickener type are guar, xanthan, cellulose gum, alginate, locust bean gum (carob bean gum) and starch, in particular potato starch, and mixtures of any of these.

A single hydrocolloid may be used, or a mixture of two or more hydrocolloids may be used. A particularly preferred mixture of hydrocolloids comprises guar, alginate, carrageenan and locust bean gum.

A preferred hydrocolloid of the gelling agent type is gelatin, in particular porcine or bovine gelatin.

The suspension of Lactic bacteria of the *coccus* type preferably has a concentration of bacteria between 50 g/kg and 200 g/kg, more preferably between 70 g/kg to 150 g/kg, based on dry weight, before addition of the hydrocolloid.

The hydrocolloid is added as a solid or as an aqueous solution or dispersion to the suspension of *coccus* bacteria, preferably below 10°C, more preferably below 5°C. For thickener hydrocolloids, after addition, the hydrocolloid is preferably present at a concentration between 0.05 wt% to 3.5 wt%, more preferably between 0.1 wt% to 1.5 wt%, particularly 0.5 wt%, 0.25 wt%, or 0.1 wt%, based on the total weight of the suspension. For gelling hydrocolloids, after addition the hydrocolloid is preferably present at a concentration between 0.25 to 4 wt%, more preferably 0.5 to 3.5 wt%, wt%, based on the total weight of the suspension. At all of these concentrations a cryoprotectant, preferably a sugar, more preferably sucrose, may additionally be added.

If alginate is used as hydrocolloid, it is preferably added at not greater than 0.3 wt%, based on the total weight of the suspension. More preferably between 0.05 and 0.3 wt%, even more preferably between 0.1 and 0.25 wt%. If starch is used as the hydrocolloid, it is preferably added at not greater than 0.2 wt%, based on the total weight of the suspension. More preferably between 0.05 and 0.1 wt%.

In preferred embodiments, the following hydrocolloids are used in the stated concentration ranges, based on the total weight of the suspension:
Guar: preferably at 0.05 to 1 wt%, more preferably at 0.25 to 0.75 wt%, particularly preferably at 0.5 wt%
Xanthan: preferably at 0.05 to 1 wt%, more preferably at 0.25 to 0.75 wt%, particularly preferably at 0.5 wt%
Cellulose gum: preferably at 0.05 to 1 wt%, more preferably at 0.25 to 0.75 wt%, particularly preferably at 0.5 wt%
Alginate: preferably at 0.05 to 0.4 wt%, more preferably at 0.1 to 0.3 wt%, particularly preferably at 0.25 wt%
Starch: preferably at 0.01 to 0.25 wt%, more preferably at 0.05 to 0.15 wt%, particularly preferably at 0.1 wt%
Gelatin: preferably 0.1 to 1 wt%, more preferably 0.25 to o.8 wt%, particularly preferably at 0.5 wt%
A mixture of guar, alginate, carrageenan and locust bean gum: preferably at 0.1 to 0.6 wt%, more preferably at 0.2 to 0.5 wt%, more preferably at 0.3 wt%.

In a further preferred embodiment, the bacteria are *Streptococcus* (in particular *Streptococcus thermophilus*) and the following hydrocolloids are used in the stated concentration ranges, based on total weight of the suspension:
Guar: preferably at 0.05 to 1 wt%, more preferably at 0.25 to 0.75 wt%, particularly preferably at 0.5 wt%
Xanthan: preferably at 0.05 to 1 wt%, more preferably at 0.25 to 0.75 wt%, particularly preferably at 0.5 wt%
Cellulose gum: preferably at 0.05 to 1 wt%, more preferably at 0.25 to 0.75 wt%, particularly preferably at 0.5 wt%
Alginate: preferably at 0.05 to 0.4 wt%, more preferably at 0.1 to 0.3 wt%, particularly preferably at 0.25 wt%
Starch: preferably at 0.01 to 0.25 wt%, more preferably at 0.05 to 0.15 wt%, particularly preferably at 0.1 wt%
A mixture of guar, alginate, carrageenan and locust bean gum: preferably at 0.1 to 0.6 wt%, more preferably at 0.2 to 0.5 wt%, more preferably at 0.3 wt%.

In a further preferred embodiment, the bacteria are *Lactococcus* (in particular *Lactococcus* lactis) and the following hydrocolloids are used in the stated concentration ranges, based on the total weight of the suspension:
Guar: preferably at 0.05 to 1 wt%, more preferably at 0.1 to 0.75 wt%, particularly preferably at 0.25 wt%
Xanthan: preferably at 0.05 to 1 wt%, more preferably at 0.1 to 0.75 wt%, particularly preferably at 0.25 wt%
Cellulose gum: preferably at 0.05 to 0.25 wt%, more preferably at 0.05 to 0.15 wt%, particularly preferably at 0.1 wt%
Alginate: preferably at 0.05 to 0.25 wt%, more preferably at 0.05 to 0.15 wt%, particularly preferably at 0.1 wt%
Starch: preferably at 0.01 to 0.4 wt%, more preferably at 0.05 to 0.3 wt%, particularly preferably at 0.25 wt%

In a preferred embodiment, in addition to the hydrocolloid, a cryoprotectant may be added to the bacterial suspension. Preferred cryoprotectants are sucrose, trehalose, maltitol, lactose, galactose, rafinose, dextrose, maltodextrins and mixtures of these, particularly preferably sucrose. When used, the cryoprotectant should preferably be present at a concentration to lead to a ratio (RB) of bacteria (dry matter) to cryoprotectant (dry matter) between 0.4 to 0.6, more preferably from 0.42 to 0.52, particularly preferably 0.44 or 0.52. Alternatively, the cryoprotectant may be present at an amount of 4.4 wt % to 20.3 wt%, more preferably 8.2 wt% to 16 wt%, based on the total weight of the suspension. A particularly preferred cryoprotectant is sucrose + maltodextrin, particularly when used at an RB of 0.52 or at a concentration of 12 wt%, based on the weight of the suspension.

In a further preferred embodiment, the bacteria are *Lactococcus lactis,* the hydrocolloid is guar, preferably at 0.05 to 1 wt%, more preferably at 0.1 to 0.75 wt%, particularly preferably at 0.25 wt%, and the RB is between 0.4 to 0.6, particularly 0.46.

In particularly preferred embodiments the bacteria are *Streptococcus,* and the hydrocolloid is selected from guar, xanthan, cellulose gum, alginate, starch and mixtures thereof. More preferably the bacteria are *Streptococcus thermophilus,* and the hydrocolloid is selected from guar, xanthan, cellulose gum, alginate, starch and mixtures thereof. More particularly preferably the bacteria are *Streptococcus thermophilus ST11688,* and the hydrocolloid is selected from guar, xanthan, cellulose gum, alginate, starch and mixtures thereof.

In further particularly preferred embodiments the bacteria are *Lactococcus,* and the hydrocolloid is selected from guar, xanthan, alginate, gelatin and mixtures thereof. More preferably the bacteria are *Lactococcus lactis,* and the hydrocolloid is selected from guar, xanthan, alginate, gelatin and mixtures thereof. More particularly preferably the bacteria are *Lactococcus lactis ssp. cremoris,* and the hydrocolloid is selected from guar, xanthan, alginate, gelatin and mixtures thereof. Even more specifically, the bacteria are *Lactococcus lactis ssp. cremoris* SC0108, and the hydrocolloid is selected from guar, xanthan, alginate, gelatin and mixtures thereof.

Other ingredients which may be present in the bacterial suspension prior to freeze-drying include, for example, yeast extract, MgSO₄, ascorbate, polysorbate 80, sodium acetate, MnSO₄, methionine, antifoam silicon, lactose.

In a preferred embodiment of the process of the invention the following steps are carried out:
(1) an aqueous suspension of Lactic bacteria of the *coccus* type is provided, preferably at a concentration of bacteria between 50 g/kg and 200 g/kg, more preferably between 70 g/kg to 150 g/kg, based on dry weight;
(2) a hydrocolloid is added;
(3) optionally a cryoprotectant, preferably a sugar, more preferably sucrose is added. The addition of the cryoprotectant may be prior to, simultaneously with or after addition of the hydrocolloid;
(4) the suspension is frozen to provide a frozen suspension; and
(5) the frozen suspension is subjected to a vacuum to cause sublimation of the water, until weight loss stops, and/or the desired water content is achieved.

Freezing may be carried out according to known methods of freeze-drying. A typical freezing temperature is -40°C, and typical coolant is Silicon oil (bazylon type).

The bacterial suspension may be frozen in any form. A common method involves freezing a layer of bacterial suspension in tray-like containers.

After freezing, the frozen suspension is subjected to a vacuum to effect primary drying, followed by secondary drying. The vacuum is typically in the range of 10Pa to 75Pa, preferably 25 Pa.

A typical freeze-drying cycle is as follows:
Freezing phase 1: coolant at -20°C for 100 minutes until suspension reaches - 15°C
Freezing phase 2: coolant at -40°C for 30 minutes until suspension reaches - 36°C
Vacuum drying (sequentially):
   1 minute at 50 microbar, coolant at -40°C
   50 minutes at 50 microbar, coolant at -30°C
   100 minutes at 190 microbar, coolant at -10°C
   610 minutes at 190 microbar, coolant at 7°C
   330 minutes at 190 microbar, coolant at 16°C
   331 minutes at 190 microbar, coolant at 25°C
   150 minutes at 40 microbar, coolant at 25°C
   10,000 minutes at 40 microbar, coolant at 10°C
   10,000 minutes at 40 microbar, coolant at 25°C

A more aggressive freeze-drying cycle is as follows:
Freezing phase 1: coolant at -20°C for 100 minutes until suspension reaches - 15°C
Freezing phase 2: coolant at -40°C for 30 minutes until the suspension reaches -36°C
Vacuum drying (sequentially):
   50 minutes at 50 microbar, coolant at -30°C
   100 minutes at 190 microbar, coolant at -10°C
   310 minutes at 190 microbar, coolant at 7°C

The presence of the at least one hydrocolloid results in more efficient water removal and higher rates of water loss by sublimation. This reduces the time necessary for the vacuum drying phase and/or permits a higher throughput because the layer of frozen suspension can be thicker, resulting in a greater mass throughput per unit time.

The result of the vacuum drying phase is a "cake" of freeze-dried composition. The use of the at least one hydrocolloid results in a better quality cake as compared to the same process without a hydrocolloid. The better quality cake is reflected in ease of release of the cake from the drying containers, resulting in less loss of freeze-dried composition, and in less cracking of the cake, which can also lead to loss of material.

The cake may be ground to a powder and stored in a dry environment to avoid water uptake.

The method of the invention typically results in a % of water sublimated that is at least 2% better, preferably at least 5% better and more particularly preferably at least 10% better than an otherwise identical sample prepared without hydrocolloid and lyophilized under similar conditions.

### Freeze-dried bacterial composition of the invention

In a further aspect, the invention provides a freeze-dried bacterial composition comprising:
(1) freeze-dried Lactic bacteria of the *coccus* type;
(2) at least one hydrocolloid.

The process of the invention results in a freeze-dried bacterial composition of the invention, which may be e.g. in cake or powder form. The freeze-dried bacterial composition provided by the process of the invention comprises freeze-dried Lactic bacteria of the *coccus* type and at least one hydrocolloid, wherein the hydrocolloid is preferably present at 0.5 to 25 wt%, based on the total weight of the composition.

The bacterial composition of the present invention may contain one species and/or strain of Lactic bacteria of the *coccus* type, a mixture of species and/or strains of Lactic bacteria of the *coccus* type. In one aspect the composition of the present invention contains one species Lactic bacteria of the *coccus* type, and, optionally, one strain of Lactic bacteria of the *coccus* type. In one aspect the composition of the present invention contains a mixture of species of Lactic bacteria of the *coccus* type, and optionally a mixture of strains of Lactic bacteria of the *coccus* type. In one aspect the composition of the present invention contains a mixture of species of Lactic bacteria of the *coccus* type.

In a further preferred embodiment the Lactic bacteria of the *coccus* type are selected from *Streptococcus, Lactococcus, Enterococcus, Oenococcus* and *Pediococcus,* and mixtures thereof. In a particularly preferred embodiment the bacteria are selected from *Streptococcus thermophilus, Lactococcus lactis* (in particular subspecies *cremoris), Lactococcus lactis lactis* and mixtures thereof.

In one preferred embodiment the Lactic bacteria of the *coccus* type, is selected from *Streptococcus thermophilus* (ST11688 strain), *Lactococcus lactis ssp. cremoris* (SC0108 strain), and mixtures thereof.

Particularly preferred hydrocolloids are guar, xanthan, cellulose gum, alginate, locust bean gum (carob bean gum) and starch, in particular potato starch, gelatin, in particular porcine or bovine gelatin, and mixtures of any of these.

Particularly preferred hydrocolloids of the thickener type are guar, xanthan, cellulose gum, alginate, locust bean gum (carob bean gum) and starch, in particular potato starch, and mixtures of any of these.

A single hydrocolloid may be present, or a mixture of two or more hydrocolloids may be present. A particularly preferred mixture of hydrocolloids comprises guar, alginate, carrageenan and locust bean gum.

A preferred hydrocolloid of the gelling agent type is gelatin, in particular porcine or bovine gelatin.

The at least one hydrocolloid is preferably present in the freeze-dried bacterial composition of the invention in an amount of 0.5 to 25 wt%, more preferably 0.72 to 20 wt%, more particularly preferably 3 to 15 wt%, based on the total weight of the composition.

In addition to freeze-dried Lactic bacteria of the *coccus* type and at least one hydrocolloid, the freeze-dried bacterial composition of the invention may additionally comprise a cryoprotectant. Preferred cryoprotectants are sucrose, trehalose, maltitol, lactose, galactose, rafinose, dextrose, maltodextrins and mixtures of these, particularly preferably sucrose, or a mixture of sucrose and maltodextrin. If present, the cryoprotectant is preferably present in an amount of from 45 to 55 wt%, more preferably 47 to 53 wt%, based on the total weight of the freeze-dried composition.

Other ingredients which may be present in the freeze-dried bacterial composition of the invention include yeast extract, MgSO₄, ascorbate, polysorbate 80, sodium acetate, MnSO₄, methionine, antifoam silicon, lactose, maltodextrins and mixtures of these.

Preferably the Lactic bacteria of the *coccus* type and the at least one hydrocolloid together make up at least 30 wt% more preferably at least 45 wt% of the freeze-dried bacterial composition of the invention, based on the total weight of the composition.

The freeze-dried bacterial composition of the invention shows a higher glass transition temperature (T_{g}) as compared to freeze-dried compositions prepared without a hydrocolloid. A higher T_{g} is desirable, as when a freeze-dried bacterial composition is heated to above its T_{g}, the composition will liberate water which results in decreased stability and viability of the bacteria. Having a higher T_{g} means the freeze-dried bacterial composition is more stable at higher temperatures than a freeze-dried composition prepared without a hydrocolloid. Preferably the freeze-dried bacterial composition of the invention has a T_{g} of greater than -5°C, more preferably greater than 15°C, more particularly preferably greater than 25°C. Alternatively, preferably the freeze-dried bacterial composition of the invention has a T_{g} that is at least 5°C, more preferably at least 10°C, even more preferably at least 20°C higher than the T_{g} of an otherwise identical composition prepared without hydrocolloid.

Furthermore, the freeze-dried bacterial composition of the invention shows a reduced water activity (a_{w}) as compared to freeze-dried bacterial compositions prepared without a hydrocolloid. Lower a_{w} means the bacterial composition will be more stable and have a longer shelf-life. Preferably the freeze-dried bacterial composition of the invention has a water activity (a_{w}) of less than 0.5, more preferably less than 0.15, more particularly preferably less than 0.1, even more particularly preferably less than 0.07. Alternatively, preferably the freeze-dried composition of the invention has an a_{w} that is at least 30%, more preferably at least 40%, even more preferably at least 45% lower than the a_{w} of an otherwise identical composition prepared without hydrocolloid.

Preferably the freeze-dried bacterial composition of the invention has a T_{g} of greater than -5°C, more preferably greater than 15°C, more particularly preferably greater than 25°C, with an a_{w} of less than 0.5.

More preferably the freeze-dried bacterial composition of the invention has a T_{g} of greater than -5°C, more preferably greater than 15°C, more particularly preferably greater than 25°C, with an a_{w} of less than 0.3.

More preferably the freeze-dried bacterial composition of the invention has a T_{g} of greater than -5°C, more preferably greater than 15°C, more particularly preferably greater than 25°C, with an a_{w} of less than 0.15.

More preferably the freeze-dried bacterial composition of the invention has a T_{g} of greater than -5°C, more preferably greater than 15°C, more particularly preferably greater than 25°C, with an a_{w} of less than 0.1.

More particularly preferably the freeze-dried bacterial composition of the invention has a T_{g} of greater than -5°C, more preferably greater than 15°C, more particularly preferably greater than 25°C, with an a_{w} of less than 0.07.

After freeze-drying, the sample is in the form of a dry cake. The freeze-dried bacterial composition of the invention releases more easily from the drying container. This results in less waste, and/or less time and effort being required in releasing the freeze-dried compositions from the drying containers.

Typical compositions of particularly preferred freeze-dried compositions of the invention are as follows:

| Typical compositions of freeze-dried bacterial compositions of the invention, by wt%, based on total weight of the freeze-dried composition, for compositions comprising coccus type bacteria, in particular *Lactococcus* and/or *Streptococcus bacteria* | | | |
|---|---|---|---|
| Hydrocolloid | Cryoprotectant (e.g. sucrose) | Bacteria | Optional additional ingredients such as maltodextrin and MgSO₄ |
| 0.36-12.72 | 15-35 | 20-45 | 5-10 |

### Particularly preferred embodiments

1. A freeze-dried bacterial composition comprising:
   (1) freeze-dried Lactic bacteria of the *coccus* type;
   (2) at least one hydrocolloid.
2. The freeze-dried bacterial composition of embodiment 1, wherein the hydrocolloid is present at 0.5 to 25 wt%, based on the total weight of the composition.
3. The freeze-dried bacterial composition of embodiment 1 or 2, wherein the hydrocolloid is present at 0.72 to 20 wt%, based on the total weight of the composition.
4. The freeze-dried bacterial composition of embodiment 1, 2 or 3, wherein the hydrocolloid is a thickener hydrocolloid or a gelling hydrocolloid.
5. The freeze-dried bacterial composition of any one preceding embodiment, wherein the hydrocolloid is selected from guar, xanthan, cellulose gum, alginate, locust bean gum, starch, gelatin and carrageenan.
6. The freeze-dried bacterial composition of any one preceding embodiment, wherein the bacteria are selected from *Streptococcus, Lactococcus, Enterococcus, Oenococcus* and *Pediococcus,* and mixtures thereof.
7. The freeze-dried bacterial composition of any one preceding embodiment, wherein the bacteria are selected from *Streptococcus thermophilus, Lactococcus lactis* (in particular subspecies *cremoris), Lactococcus lactis lactis* and mixtures thereof.
8. The freeze-dried bacterial composition of any one preceding embodiment, wherein the composition additionally comprises a cryoprotectant.
9. The freeze-dried bacterial composition of any one preceding embodiment, wherein the composition additionally comprises a cryoprotectant selected from sucrose, trehalose, maltitol, lactose, galactose, rafinose, dextrose, maltodextrins and mixtures of these.
10. The freeze-dried bacterial composition of any one preceding embodiment, wherein the composition additionally comprises a cryoprotectant selected from sucrose and a mixture of sucrose and maltodextrin.
11. The freeze-dried bacterial composition of any one preceding embodiment, wherein the composition additionally comprises a cryoprotectant in an amount of from 45 to 55 wt%, based on the total weight of the freeze-dried composition.
12. The freeze-dried bacterial composition of any one preceding embodiment, wherein the Lactic bacteria of the *coccus* type and the hydrocolloid together make up at least 30 wt% of the composition, based on the total weight of the composition.
13. The freeze-dried bacterial composition of any one preceding embodiment, having a T_{g} of greater than -5°C.
14. The freeze-dried bacterial composition of any one preceding embodiment, having a T_{g} of greater than 20°C.
15. The freeze-dried bacterial composition of any one preceding embodiment, having a water activity (a_{w}) of less than 0.5.
16. The freeze-dried bacterial composition of any one preceding embodiment, having a water activity (a_{w}) of less than 0.3.
17. The freeze-dried bacterial composition of any one preceding embodiment, having a T_{g} of greater than 15°C and an a_{w} of less than 0.5.
18. The freeze-dried bacterial composition of any one preceding embodiment, having a T_{g} of greater than 15°C and an a_{w} of less than 0.15.
19. The freeze-dried bacterial composition of any one preceding embodiment, having a T_{g} of greater than 15°C and an a_{w} of less than 0.07.
20. The freeze-dried bacterial composition of any one preceding embodiment, wherein the bacteria are *Streptococcus thermophilus,* and the hydrocolloid is selected from guar, xanthan, cellulose gum, alginate, starch, carrageenan, locust bean gum and mixtures of any of these.
21. The freeze-dried bacterial composition of any one preceding embodiment, wherein the bacteria are *Streptococcus thermophilus,* and the hydrocolloid is a mixture of guar, alginate, carrageenan and locust bean gum.
22. The freeze-dried bacterial composition of any one preceding embodiment, wherein the bacteria are *Lactococcus lactis,* and the hydrocolloid is selected from guar, xanthan, alginate, gelatin and mixtures thereof.
23. The freeze-dried bacterial composition of any one preceding embodiment, wherein the bacteria are present at from 20-45 wt%, and the hydrocolloid is present at from 0.36-12.75 wt%, based on the total weight of the freeze-dried composition.
24. The freeze-dried bacterial composition of any one preceding embodiment, comprising 15-35 wt% cryoprotectant, for example, sucrose, based on the total weight of the freeze-dried composition.
25. The freeze-dried bacterial composition of any one preceding embodiment, in the form of a cake or powder.
26. A method for preparing lyophilized Lactic bacteria of the *coccus* type, comprising the step of adding a hydrocolloid to an aqueous suspension of Lactic bacteria of the *coccus* type before freezing and freeze-drying.
27. The method of embodiment 26, wherein the hydrocolloid is a thickener hydrocolloid or a gelling hydrocolloid.
28. The method of embodiment 26 or 27, wherein the hydrocolloid is selected from guar, xanthan, cellulose gum, alginate, starch, gelatin and carrageenan.
29. The method of embodiment 26, 27 or 28, wherein the hydrocolloid is added to the aqueous suspension of *coccus* bacteria to yield a solution having a concentration of 0.1 to 3.5 of hydrocolloid, based on the total weight of the solution.
30. The method of any one of embodiments 26 to 29, wherein the addition of hydrocolloid is made to a suspension of bacteria having a bacterial concentration between 50 g/kg and 200 g/kg, more preferably between 70 g/kg to 150 g/kg, based on dry weight.
31. The method of any one of embodiments 26 to 30, wherein a cryoprotectant is added to the aqueous suspension of *coccus* bacteria.
32. The method of embodiment 31, wherein the cryoprotectant is selected from sucrose, trehalose, maltitol, lactose, galactose, rafinose, dextrose, maltodextrins and mixtures of these.
33. The method of embodiment 31, wherein the cryoprotectant is sucrose.
34. The method of embodiment 31, 32 or 33, wherein the cryoprotectant is added to yield an RB of from 0.44 to 0.52.
35. The method of any one of embodiments 26 to 34, wherein the cryoprotectant is added to the suspension at a concentration between 4.4 to 20.3 wt%, based on the total weight of the suspension.
36. The method of any one of embodiments 26 to 35, wherein the bacteria are selected from *Streptococcus, Lactococcus, Enterococcus, Oenococcus* and *Pediococcus,* and mixtures thereof.
37. The method of any one of embodiments 26 to 36, wherein the bacteria are selected from *Streptococcus, Lactococcus* and mixtures thereof.
38. The method of any one of embodiments 26 to 37, wherein the bacteria are selected from *Streptococcus thermophilus, Lactococcus lactis* (in particular subspecies *cremoris*)*, Lactococcus lactis lactis* and mixtures thereof.
39. The method of any one of embodiments 26 to 38, wherein the bacteria are *Streptococcus thermophilus,* and the hydrocolloid is selected from guar, xanthan, cellulose gum, alginate and starch.
40. The method of any one of embodiments 26 to 38, wherein the bacteria are *Lactococcus lactis,* and the hydrocolloid is selected from guar, xanthan, alginate, gelatin and mixtures thereof.
41. A freeze-dried bacterial composition obtainable by the method of any one of embodiments 26 to 40.

### EXAMPLES

### Materials

The following bacterial strains were used:
*Streptococcus thermophilus* ST11688 strain
*Lactococcus lactis ssp. cremoris* SC0108 strain
*Lactobacillus bulgaricus* LB0034 strain

The following hydrocolloids were used:
Guar (Grindsted Guar 175)
Xanthan (Grindsted Xanthan Supra)
Cellulose gum (Grindsted)
Sodium alginate (Grindsted Alginate PH 175)
Potato starch (Pregeflo M)
Bovine Gelatin
Carrageenan
Locust bean gum

### Analytical Methods

The progress of lyophilization was followed by monitoring the temperature of the sample.

The quantity of sublimated water was measured by weighing the sample before and after lyophilization. Dividing the mass of water lost by the time of sublimation yielded the average rate of sublimation.

### Samples

Samples were prepared as follows:
Bacterial suspensions were prepared by ultrafiltration of fermentation broths to a concentration of 50 g/kg. To the bacterial suspensions at 4°C were added dispersions of the hydrocolloid and sucrose in the quantities shown in Tables 1, 2 and 3. Table 3 shows the composition of comparative examples in which bacteria of non-coccus-type were used (*Lactobacillus bulgaricus*)*.*

| **Table 1. Samples prepared for examples and comparative examples using *Streptococcus thermophilus* ST11688** | | | | |
|---|---|---|---|---|
| **Example no.** | **Hydrocolloid** | **RB** | **Wt% hydrocolloid** | **Wt% sucrose** |
| C1 | None | 0.52 | 0 | 8,1 |
| EX1a | Guar | 0.46 | 0.25 | 10,0 |
| EX1b | Guar | 0.52 | 0.1 | 8,1 |
| EX1c | Guar | 0.52 | 0.25 | 8,0 |
| EX1d | Guar | 0.52 | 0.5 | 8,0 |
| EX2a | Xanthan | 0.52 | 0.1 | 8,1 |
| EX2b | Xanthan | 0.52 | 0.25 | 8,0 |
| EX2c | Xanthan | 0.52 | 0.5 | 8,0 |
| EX3a | Cellulose gum | 0.52 | 0.1 | 8,1 |
| EX3b | Cellulose gum | 0.52 | 0.25 | 8,0 |
| EX3c | Cellulose gum | 0.52 | 0.5 | 8,0 |
| EX4a | Alginate | 0.52 | 0.1 | 8,1 |
| EX4b | Alginate | 0.52 | 0.25 | 8,0 |
| EX4c | Alginate | 0.52 | 0.5 | 8,0 |
| EX5a | Starch | 0.52 | 0.1 | 8,1 |
| EX5b | Starch | 0.52 | 0.25 | 8,0 |
| EX5c | Starch | 0.52 | 0.5 | 8,0 |

| **Table 2. Samples prepared for examples and comparative example using *Lactococcus lactis ssp. cremoris* SC0108** | | | | |
|---|---|---|---|---|
| **Example no.** | **Hydrocolloid** | **RB** | **Wt% hydrocolloid** | **Wt% sucrose** |
| C2 | None | 0.52 | 0 | 4,6 |
| EX6a | Guar | 0.46 | 0.25 | 5,7 |
| EX6b | Guar | 0.52 | 0.1 | 4,6 |
| EX6c | Guar | 0.52 | 0.25 | 4,6 |
| EX6d | Guar | 0.52 | 0.5 | 4,6 |
| EX7a | Xanthan | 0.52 | 0.1 | 4,6 |
| EX7b | Xanthan | 0.52 | 0.25 | 4,6 |
| EX7c | Xanthan | 0.52 | 0.5 | 4,6 |
| EX8a | Cellulose gum | 0.52 | 0.1 | 4,6 |
| EX8b | Cellulose gum | 0.52 | 0.25 | 4,6 |
| EX8c | Cellulose gum | 0.52 | 0.5 | 4,6 |
| EX9a | Alginate | 0.52 | 0.1 | 4,6 |
| EX9b | Alginate | 0.52 | 0.25 | 4,6 |
| EX9c | Alginate | 0.52 | 0.5 | 4,6 |
| EX10a | Starch | 0.52 | 0.1 | 4,6 |
| EX10b | Starch | 0.52 | 0.25 | 4,6 |
| EX10c | Starch | 0.52 | 0.5 | 4,6 |
| EX11a | Gelatin | 0.52 | 0.5 | 4,6 |
| EX11b | Gelatin | 0.52 | 2.5 | 4,5 |
| EX12a | Carrageenan | 0.52 | 3.5 | 4,5 |

| **Table 3. Samples prepared for comparative examples using *Lactobacillus bulgaricus* LB0034** | | | | |
|---|---|---|---|---|
| **Example no.** | **Hydrocolloid** | **RB** | **Wt% hydrocolloid** | **Wt% sucrose** |
| C3 | None | 0.52 | 0 | 8,3 |
| C4a | Guar | 0.46 | 0.25 | 10,2 |
| C4b | Guar | 0.52 | 0.1 | 8,3 |
| C4c | Guar | 0.52 | 0.25 | 8,3 |
| C4d | Guar | 0.52 | 0.5 | 8,3 |
| C5a | Xanthan | 0.52 | 0.1 | 8,3 |
| C5b | Xanthan | 0.52 | 0.25 | 8,3 |
| C5c | Xanthan | 0.52 | 0.5 | 8,3 |
| C6a | Cellulose gum | 0.52 | 0.1 | 8,3 |
| C6b | Cellulose gum | 0.52 | 0.25 | 8,3 |
| C6c | Cellulose gum | 0.52 | 0.5 | 8,3 |
| C7a | Alginate | 0.52 | 0.1 | 8,3 |
| C7b | Alginate | 0.52 | 0.25 | 8,3 |
| C7c | Alginate | 0.52 | 0.5 | 8,3 |
| C8a | Starch | 0.52 | 0.1 | 8,3 |
| C8b | Starch | 0.52 | 0.25 | 8,3 |
| C8c | Starch | 0.52 | 0.5 | 8,3 |

### Freeze-drying

The liquid samples were poured/filled into lyophilizing trays and subjected to the following freezing and freeze-drying cycle:
Freezing phase 1: coolant at -20°C for 100 minutes until suspension reached - 15°C
Freezing phase 2: coolant at -40°C for 30 minutes until the suspension reached -36°C
Vacuum drying (sequentially):
   50 minutes at 50 microbar, coolant at -30°C
   100 minutes at 190 microbar, coolant at -10°C
   310 minutes at 190 microbar, coolant at 7°C
Vacuum broken at 7°C

The weight of the samples before and after drying was measured and used to calculate the percentage of water sublimated. The amount of water sublimated was divided by the time required to give a sublimation rate. The results are listed in Tables 4, 5 and 6.

| **Table 4. Percent water sublimated and rate of sublimation for *Streptococcus thermophilus* ST11688 in the presence of different hydrocolloids** | | | | | | |
|---|---|---|---|---|---|---|
| **Example no.** | **Hydrocolloid (wt%)** | **RB** | **% of water sublimated** | **Δ VS control** | **Rate of sublimation (g/hour)** | **Δ VS control** |
| C1 | None | 0.52 | 26.5 | 0 | 68.3 | 0 |
| EX1a | 0.25 Guar | 0.46 | 31.6 | +5.1 | 79.4 | +11.1 |
| EX1b | 0.1 Guar | 0.52 | 29.2 | +2.7 | 75.0 | +6.7 |
| EX1c | 0.25 Guar | 0.52 | 34.1 | +7.6 | 87.4 | +19.1 |
| EX1d | 0.5 Guar | 0.52 | 42.2 | +15.7 | 108.6 | +40.3 |
| EX2a | 0.1 Xanthan | 0.52 | 37.5 | +11.0 | 96.3 | +28.0 |
| EX2b | 0.25 Xanthan | 0.52 | 33.4 | +6.9 | 82.8 | +14.5 |
| EX2c | 0.5 Xanthan | 0.52 | 39.2 | +12.7 | 100.6 | +32.3 |
| EX3a | 0.1 Cellulose gum | 0.52 | 31.1 | +4.6 | 79.9 | +11.6 |
| EX3b | 0.25 Cellulose gum | 0.52 | 41.9 | +15.4 | 107.2 | +38.9 |
| EX3c | 0.5 Cellulose gum | 0.52 | 43.1 | +16.6 | 110.6 | +42.3 |
| EX4a | 0.1 Alginate | 0.52 | 26.9 | +0.4 | 68.8 | +0.5 |
| EX4b | 0.25 Alginate | 0.52 | 36.0 | +9.5 | 92.7 | +24.4 |
| EX4c | 0.5 Alginate | 0.52 | 22.8 | -3.7 | 58.6 | -9.7 |
| EX5a | 0.1 Starch | 0.52 | 40.1 | +13.6 | 103.0 | +34.7 |
| EX5b | 0.25 Starch | 0.52 | 27.3 | +0.8 | 69.9 | +1.6 |
| EX5c | 0.5 Starch | 0.52 | 25.6 | -0.9 | 65.4 | -2.9 |

| **Table 5. Percent water sublimated and rate of sublimation for *Lactococcus lactis ssp. cremoris SC0108* in the presence of different hydrocolloids** | | | | | | |
|---|---|---|---|---|---|---|
| **Example no.** | **Hydrocolloid (wt%)** | **RB** | **% of water sublimated** | **Δ VS control** | **Rate of sublimation (g/hour)** | **Δ** VS **control** |
| C2 | None | 0.52 | 24.7 | 0 | 54.4 | 0 |
| EX6a | 0.25 Guar | 0.46 | 37.2 | +12.5 | 80.3 | +25.9 |
| EX6b | 0.1 Guar | 0.52 | 28.1 | +3.4 | 61.3 | +6.9 |
| EX6c | 0.25 Guar | 0.52 | 29.9 | +5.2 | 65.8 | +11.4 |
| EX6d | 0.5 Guar | 0.52 | 32.2 | +7.5 | 70.4 | +16.0 |
| EX7a | 0.1 Xanthan | 0.52 | 25.9 | +1.2 | 57.2 | +2.8 |
| EX7b | 0.25 Xanthan | 0.52 | 33.6 | +8.9 | 74.1 | +19.7 |
| EX7c | 0.5 Xanthan | 0.52 | 30.5 | +5.8 | 66.9 | +12.5 |
| EX8a | 0.1 Cellulose gum | 0.52 | 26.9 | +2.2 | 59.2 | +4.8 |
| EX8b | 0.25 Cellulose gum | 0.52 | 22.2 | -2.5 | 49.0 | -5.4 |
| EX8c | 0.5 Cellulose gum | 0.52 | 23.9 | -0.8 | 53.5 | -0.9 |
| EX9a | 0.1 Alginate | 0.52 | 31.7 | +7.0 | 69.8 | +15.4 |
| EX9b | 0.25 Alginate | 0.52 | 27.3 | +2.6 | 60.6 | +6.2 |
| EX9c | 0.5 Alginate | 0.52 | 25.5 | +0.8 | 56.1 | +1.7 |
| EX10a | 0.1 Starch | 0.52 | 27.4 | +2.7 | 60.3 | +5.9 |
| EX10b | 0.25 Starch | 0.52 | 27.6 | +2.9 | 61.0 | +6.6 |
| EX10c | 0.5 Starch | 0.52 | 24.9 | +0.2 | 54.8 | +0.4 |
| EX11a | 0.5 Gelatin | 0.52 | 35.1 | +10.4 | 77.6 | +23.2 |
| EX11b | 2.5 Gelatin | 0.52 | 27.0 | +2.3 | 58.5 | +4.1 |
| EX12a | 3.5 Carrageenan | 0.52 | 26.1 | +1.4 | 59.4 | +5.0 |

| **Table 6. Percent water sublimated and rate of sublimation for *Lactobacillus bulgaricus LB0034* in the presence of different hydrocolloids** | | | | | | |
|---|---|---|---|---|---|---|
| **Example no.** | **Hydrocolloid (wt%)** | **RB** | **% of water sublimated** | **Δ VS control** | **Rate of sublimation (g/hour)** | **Δ VS control** |
| C3 | None | 0.52 | 25.3 | 0 | 57.0 | 0 |
| C4a | 0.25 Guar | 0.46 | 23.1 | -2.2 | 51.2 | -5.8 |
| C4b | 0.1 Guar | 0.52 | 22.0 | -3.3 | 49.6 | -7.4 |
| C4c | 0.25 Guar | 0.52 | 23.6 | -1.7 | 53.4 | -3.6 |
| C4d | 0.5 Guar | 0.52 | 21.3 | -4.0 | 48.2 | -8.8 |
| C5a | 0.1 Xanthan | 0.52 | 26.0 | +0.7 | 58.9 | +1.9 |
| C5b | 0.25 Xanthan | 0.52 | 24.4 | -0.9 | 55.0 | -2.0 |
| C5c | 0.5 Xanthan | 0.52 | 24.9 | -0.4 | 56.3 | -0.7 |
| C6a | 0.1 Cellulose gum | 0.52 | 21.7 | -3.6 | 49.2 | -7.8 |
| C6b | 0.25 Cellulose gum | 0.52 | 19.9 | -5.4 | 44.0 | -13 |
| C6c | 0.5 Cellulose gum | 0.52 | 24.4 | -0.9 | 55.2 | -1.8 |
| C7a | 0.1 Alginate | 0.52 | 22.1 | -3.2 | 49.6 | -7.4 |
| C7b | 0.25 Alginate | 0.52 | 19.4 | -5.9 | 43.6 | -13.4 |
| C7c | 0.5 Alginate | 0.52 | 22.6 | -2.7 | 50.8 | -6.2 |
| C8a | 0.1 Starch | 0.52 | 22.1 | -3.2 | 50.7 | -6.3 |
| C8b | 0.25 Starch | 0.52 | 25.7 | +0.4 | 57.9 | +0.9 |
| C8c | 0.5 Starch | 0.52 | 21.0 | -4.3 | 47.3 | -9.7 |

It is clear from Tables 4, 5 and 6 that the presence of hydrocolloid increases the percentage of water sublimated and increases the rate of sublimation for Lactic bacteria of the *coccus* type (*Streptococcus thermophilus* and *Lactococcus lactis*) whereas there is no improvement or even a decrease in these parameters for non-coccus bacteria (*Lactobacillus bulgaricus*)*.*

The resulting cakes were released from the trays and ground to produce powdered lyophilized bacterial compositions which were stored under dry conditions.

### Water activity

Samples comprising *Streptococcus thermophilus* (ST11688 strain) were freeze-dried as above, using different concentrations of hydrocolloid, and sucrose at an RB of 0.48. The hydrocolloid used was a mixture of guar, alginate, carrageenan and locust bean gum. The comparative sample (no hydrocolloid) and the three samples containing hydrocolloid are designated C9 and EX13, EX14 and EX15, respectively.

Water activity (a_{w}) was measured after freeze-drying and compared to a sample prepared otherwise identically, but without the hydrocolloid. The results are shown in Table 7.

| **Table 7. Water activity (a_{w}) for freeze-dried *Streptococcus thermophilus* in the presence of different concentrations of hydrocolloid mixture (guar, alginate, carrageenan and locust bean gum)** | | |
|---|---|---|
| **Example No.** | **Concentration of hydrocolloid (wt%) (based on total weight of suspension)** | **a_{w}** |
| C9 | 0 | 0.5 |
| EX13 | 0.2 | 0.3 |
| EX14 | 0.3 | 0.25 |
| EX15 | 0.4 | 0.28 |

The data in Table 7 shows that the use of a hydrocolloid according to the invention leads to a reduced water activity. Use of hydrocolloid at 0.3 wt% (EX14) gives the best results for a_{w}. Reduced water activity is directly correlated to increased shelf-life of the freeze-dried composition.

### Glass transition temperature (T_{g})

The T_{g} was also measured for freeze-dried samples C9, EX13, EX14 and EX15, listed in above. The results are listed in Table 8.

| **Table 8. Glass transition temperature (T_{g}) for freeze-dried *Streptococcus thermophilus* in the presence of different concentrations of hydrocolloid mixture (guar, alginate, carrageenan and locust bean gum)** | | |
|---|---|---|
| **Example No.** | **Concentration of hydrocolloid (wt%) (based on total weight of suspension)** | **T_{g} (°C)** |
| C9 | 0 | -4.4 |
| EX13 | 0.2 | 20 |
| EX14 | 0.3 | 29.1 |
| EX15 | 0.4 | 25.3 |

The results in Table 8 show that the use of a hydrocolloid according to the invention results in a significantly increased T_{g}. Use of hydrocolloid at 0.3 wt% (EX14) gives the best results for T_{g}. A higher T_{g} is desirable, as when a freeze-dried bacterial composition is heated to above its T_{g}, the composition will liberate water which results in decreased stability and viability of the bacteria. Having a higher T_{g} means the freeze-dried bacterial composition is more stable at higher temperatures than a freeze-dried composition prepared without a hydrocolloid.

## Claims

1. A freeze-dried bacterial composition comprising:
(1) freeze-dried Lactic bacteria of the *coccus* type;
(2) at least one hydrocolloid.

2. The freeze-dried bacterial composition of claim 1, wherein the hydrocolloid is present at 0.5 to 25 wt%, based on the total weight of the composition.

3. The freeze-dried bacterial composition of claim 1 or 2, wherein the hydrocolloid is present at 0.72 to 20 wt%, based on the total weight of the composition.

4. The freeze-dried bacterial composition of any one preceding claim, wherein the hydrocolloid is selected from guar, xanthan, cellulose gum, alginate, locust bean gum, starch, gelatin and carrageenan.

5. The freeze-dried bacterial composition of any one preceding claim, wherein the bacteria are selected from *Streptococcus, Lactococcus, Enterococcus, Oenococcus* and *Pediococcus,* and mixtures thereof.

6. The freeze-dried bacterial composition of any one preceding claim, wherein the bacteria are selected from *Streptococcus thermophilus, Lactococcus lactis* (in particular subspecies *cremoris*)*, Lactococcus lactis lactis* and mixtures thereof.

7. The freeze-dried bacterial composition of any one preceding claim, wherein the composition additionally comprises a cryoprotectant selected from sucrose, trehalose, maltitol, lactose, galactose, rafinose, dextrose, maltodextrins and mixtures of these.

8. The freeze-dried bacterial composition of any one preceding claim, wherein the Lactic bacteria of the *coccus* type and the hydrocolloid together make up at least 30 wt% of the composition, based on the total weight of the composition.

9. The freeze-dried bacterial composition of any one preceding claim, having a T_{g} of greater than -5°C.

10. The freeze-dried bacterial composition of any one preceding claim, having a water activity (a_{w}) of less than 0.5.

11. The freeze-dried bacterial composition of any one preceding claim, having a T_{g} of greater than 15°C and an a_{w} of less than 0.5.

12. The freeze-dried bacterial composition of any one preceding claim, wherein the bacteria are *Streptococcus thermophilus,* and the hydrocolloid is selected from guar, xanthan, cellulose gum, alginate, starch, carrageenan, locust bean gum and mixtures of any of these.

13. The freeze-dried bacterial composition of any one preceding claim, wherein the bacteria are *Lactococcus lactis,* and the hydrocolloid is selected from guar, xanthan, alginate, gelatin and mixtures thereof.

14. A method for preparing lyophilized Lactic bacteria of the *coccus* type, comprising the step of adding a hydrocolloid to an aqueous suspension of Lactic bacteria of the *coccus* type before freezing and freeze-drying.

15. The method of claim 14, wherein the hydrocolloid is selected from guar, xanthan, cellulose gum, alginate, starch, gelatin and carrageenan.

16. The method of claim 14 or 15, wherein the hydrocolloid is added to the aqueous suspension of *coccus* bacteria to yield a solution having a concentration of 0.1 to 3.5 of hydrocolloid, based on the total weight of the solution.

17. The method of any one of claims 14 to 16, wherein the addition of hydrocolloid is made to a suspension of bacteria having a bacterial concentration between 50 g/kg and 200 g/kg, more preferably between 70 g/kg to 150 g/kg, based on dry weight.

18. The method of any one of claims 14 to 17, wherein the bacteria are selected from *Streptococcus, Lactococcus, Enterococcus, Oenococcus* and *Pediococcus,* and mixtures thereof.

19. The method of any one of claims 14 to 18, wherein the bacteria are selected from *Streptococcus, Lactococcus* and mixtures thereof.

20. The method of any one of claims 14 to 19, wherein the bacteria are selected from *Streptococcus thermophilus, Lactococcus lactis* (in particular subspecies *cremoris), Lactococcus lactis lactis* and mixtures thereof.

21. The method of any one of claims 14 to 20, wherein the bacteria are *Streptococcus thermophilus,* and the hydrocolloid is selected from guar, xanthan, cellulose gum, alginate and starch.

22. The method of any one of claims 14 to 20, wherein the bacteria are *Lactococcus lactis,* and the hydrocolloid is selected from guar, xanthan, alginate, gelatin and mixtures thereof.

23. A freeze-dried bacterial composition obtainable by the method of any one of claims 14 to 22.
